# EUROPEAN PATENT SPECIFICATION

(11) **EP 1 918 712 B1**
(45) Date of publication and mention of the grant of the patent: **24.03.2010**
(21) Application number: 08000375.9
(22) Date of filing: 18.09.2002
(51) Int. Cl.: G01N 33/58

(54) **System for the detection of urease and method for using same**
System zur Erkennung von Urease und Verfahren zur Verwendung desselben
Système de détection d'urée et procédé d'utilisation

(30) Priority: 15.10.2001 US 977555; 15.10.2001 US 977556; 15.10.2001 US 977874; 15.10.2001 US 977667
(43) Date of publication of application: 07.05.2008
(62) Divisional of application: 02763668.7
(73) Proprietor: Marshall, Barry, Subiaco 6904 (AU)
(72) Inventor: McMichael, Donald, J., South Jordan Utah 84095 (US); Peterson, Kristy, Salt Lake City Utah 84117 (US); Marshall, Barry, J., Dalkeith Western Australia 6009 (AU); Mendis, Aruni, H., W., Connolly Western Australia 6027 (AU); Chairman, Simon, Beaconsfield Victoria 3807 (AU)
(74) Representative: Grünecker, Kinkeldey, Stockmair & Schwanhäusser Anwaltssozietät

(56) References cited:
- EP-A- 0 896 547
- US-A- 4 748 113
- US-A- 5 782 951

## Description

### Background of the Invention

Many ailments of the gastrointestinal system in humans are caused at least in part by bacteria. Such bacteria include those of the genus *Campylobacter*, and particularly *Helicobacter pylori*. For example, *Helicobacter pylori* can cause bacterial infections on the mucosal surface of the gastrointestinal tract, particularly on the surface of the stomach. The chronic disorders of the gastrointestinal system that can be caused by bacteria include chronic or atrophic gastritis, gastroenteritis, non-ulcer dyspepsia, esophageal reflux disease, gastric motility disorders, peptic ulcers including gastric and duodenal ulcers, and the like.

Once a patient is showing symptoms of a gastrointestinal disorder, several tests can be used to diagnose the disorder, including the diagnosis of a possible bacterial infection. Diagnostic testing systems have been manufactured to test for a wide variety of conditions in numerous types of samples, such as, for example, blood, tissue biopsies, and saliva. Such testing systems may be utilized to determine the presence of particular bacteria, such as *Helicobacter pylori*. Some tests that have been proposed to detect *Helicobacter pylori* include those that are disclosed in numerous U.S. Patents, including, for example, U.S. Patent No. 4,748,113 to Marshall, U.S. Patent No. 5,314,804 to Boguslaski et al., U.S Patent No. 5,439,801 to Jackson, U.S. Patent No. 5,702,911 to Whalen, U.S. Patent No. 5,989,840 to D'Angelo et al., U.S. Patent No. 6,068,985 to Cripps et al., U.S. Patent No. 6,156,346 to Chen et al., and U.S. Patent No. 6,187,556 to Lee et al., each of such patents being incorporated in their entirety by reference herein.

*Helicobacter pylori* produces an enzyme called urease. Various tests detect the presence of urease on a sample, such as, for example, a gastric sample that is obtained through endoscopy. In the tests described above, other biological samples may be used, such as, for example, blood, saliva, or urine. Urease is known to convert urea into ammonium carbonate, which then decomposes into ammonia and carbon dioxide. Consequently, in the past, one test for detecting the presence of *Helicobacter pylori* included the steps of contacting a sample of gastric material with a composition containing urea and an indicator, namely a pH indicator that changes color when there is a rise in pH. If urease is present within the gastric material it breaks down the urea, which results in the formation of ammonia after further decomposition and causes the pH indicator to change color.

The gastric material that is collected from the patient is typically a biopsy specimen that is removed from the gastric mucosa at endoscopy by means of biopsy forceps, Typically, the tissue sample is inserted into a gel that contains urea and the indicator.

Although the above method has provided great advancements in the early detection of gastrointestinal disorders, the tasting composition used to detect the presence of urease has a limited shelf life. In particular, the urea and other reagents contained within the composition can have a tendency to degrade over time. Consequently, once formulated, the testing composition should be used In a relatively short amount of time and is also typically refrigerated prior to use In order to prevent degradation.

US -A- 5 782 951 discloses particulate urea employed in fertilizer industry by application to the soil or as an intermediate product for the subsequent incorporation with other fertilizer material into solid bulk blends.

US -A- 4 748 113 discloses a composition comprising urea, bactericide and a pH indicator for the detection of grastrointestinal disorders.

EP -A- 0 896 547 discloses discloses the manufacture of urea in a tablet form that allows for detection of infection of the stomach (see page 5, line 14 to page 6, line 18). The active substance urea is provided in a fine particulate form with a particle size of below 20 mm.

In view of the above, a need currently exists for an improved testing composition and associated method for the detection of bacterial infections in the gastrointestinal tract of patients. More particularly, a need exists for a composition for detecting urease in gastric samples that has a prolonged shelf life.

### Summary of the Invention

The present invention is directed to a material well suited for detecting the presence of urease in a gastric material for diagnosing gastrointestinal disorders. The material includes a composition comprising a powder, said composition including urea and an anti-caking agent, said urea having a mean particle size of less than about 0.01 mm, wherein said anti-caking agent comprises sodium alumino silicate or silica having a particle size not greater than the particle size of said urea.

Other features and advantages of the present invention will be discussed in greater detail below.

### Brief Description of the Drawings

The present invention is illustrated with reference to the accompanying figures, in which:
Figure 1 is a perspective view of a system for detecting urease;
Figure 2 is a top view of the system illustrated in Figure 1;
Figure 3 is a cross-sectional view of the system illustrated In Figure 1;
Figure 4 is a cross-sectional view of another urease testing device.
Figure 5 is a perspective view of a system, container and specimen-handling tool;
Figure 6 is a perspective view of a container;
Figure 7 is a perspective view of the bottom of a container;
Figure 8 is a side view of a container;
Figure 9 is a top view of another container;
Figure 10 is a perspective view of a specimen-handling tool;
Figure 11 is a side view of the specimen-handling tool depicted in Figure 10;
Figure 12 is another perspective view of a specimen-handling tool;
Figure 13 is a top view of the specimen-handling tool that is depicted in Figure 12;
Figure 14 is a perspective view of yet another specimen-handling tool;
Figure 15 is a perspective view of still another specimen-handling tool;
Figure 16 is a perspective view of another system, carrier and specimen-handling tool;
Figure 17 is a cross-sectlonal view of the example depicted in Figure 16, taken along line 13-13;
Figure 18 is a perspective cross-sectional view of the example depicted in Figure 16, taken along line 14-14;
Figure 19 is a perspective view of a system;
Figure 20 is a cross-sectional view of the example depicted in Figure 18, taken along line 16-16; and
Figure 21 is a perspective view of another specimen-handling tool.

Repeated use of reference characters in the present specification and drawings is Intended to represent same or analogous features or elements of the invention.

### Detailed Description of Embodiments

The present invention is generally directed to the detection of the presence of urease on a gastric biopsy sample. Urease is an enzyme known to be produced by bacteria that are harmful to the gastrointestinal tract, including bacteria such as *Helicobacter pylori*. Gastrointestinal disorders that can be caused by bacterial infections include chronic or atrophic gastritis, gastroenteritis, non-ulcer dyspepsia, esophageal reflux disease, gastric motility disorders, peptic ulcers including gastric and duodenal ulcers, and the like.

In the past, In order to detect bacterial infections in the gastrointestinal tract, a biopsy sample of gastric material was first obtained. The biopsy sample was then contacted with a composition containing urea and an indicator, such as a pH indicator. If urease were present in the biopsy sample, the urease would break down and convert the urea In the composition to ammonia subsequently causing a rise In the pH of the composition. The rise in pH then caused the indicator to undergo a color change.

As described above, however, the composition containing urea and the indicator has a relatively short shelf life due to the Instability of various ingredients in the composition, including the urea. The present invention is directed to an improved test for gastrointestinal disorders caused by bacterial infections. According to the present invention, in order to improve the shelf life of systems and devices designed to detect bacterial infections in the gastrointestinal tract, a composition containing urea is separated from a composition containing an indicator. The two compositions are then sequentially contacted with a biopsy sample in order to detect the presence of urease.

More particularly, the first composition contains urea in a finely powdered, dry state. By maintaining urea in a powdered form separate from the agar and the indicator, the urea remains more stable. Further, by maintaining the urea separate from the indicator, the handling requirements of the test system become more relaxed. For instance, by maintaining both compositions separate, there is no need to refrigerate the compositions prior to use or during shipping.

By maintaining the urea separate from the indicator composition, the process conditions for manufacturing the indicator composition also become relaxed. In particular, the indicator composition, such as an indicator gel, is much more stable during manufacture, allowing larger batches to be produced that are not sensitive to ingredients contained within the composition and to temperature variations.

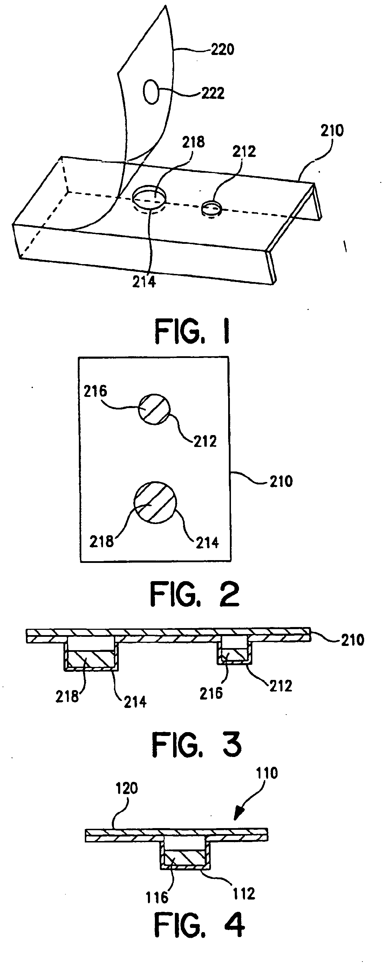

The system for detecting urease can come in many forms, and for purposes of explanation Figures 1-3 illustrate a device for detecting urease in biopsy samples. As shown, the testing device includes a single container 210 defining a first well 212 and a second well 214. Contained in the first well 212 Is a first composition 216 containing urea, such as urea In a finely powdered state with an anti-caking agent.

In the second well 214, on the other hand, Is a second composition 218 containing an indicator. The indicator is configured to detect the presence of ammonia.

In this embodiment, the device 210 further includes a removable top 220 that covers the first well 212 and the second well 214. For example, the top 220 can be made from a plastic film. The top 220 Is provided in order to prevent the first composition 216 or the second composition 218 from spilling or becoming contaminated prior to use.

In order to protect the powdered urea, the film top 220 can be made liquid impermeable at a location over the first well 212. In particular, the entire film top 220 can be liquid impermeable or, alternatively, a separate membrane 222 as shown in Figure 1 can be placed over the first well 212 that is liquid impermeable.

Besides or in addition to making the top film 220 liquid impermeable, the membrane 222 can also be used to prevent urea particles from sticking to the film top when the film top is removed.

In order to perform a urease test using the device shown in Figure 1, a biopsy sample is first taken from the lining of the gastrointestinal tract of a patient, such as from the lining of the stomach. The biopsy sample can be taken at endoscopy using biopsy forceps. The top film 220 is peeled back to expose the first composition 216 in the first well 212. The biopsy sample is then contacted with the first composition causing the urea powder to stick to the sample. For example, the biopsy sample can be rolled in the first composition much like the process of "flouring" a food product prior to cooking.

Once the first composition has coated the biopsy sample, the sample is then contacted with the second composition 218 containing an indicator located in the second well 214. Once contacted with the second composition, the powdered urea on the surface of the biopsy sample is moistened and activated by the second composition. Once moistened, the urea powder becomes available in greater amounts to any urease enzyme present in the biopsy sample. If present, the urease converts the urea into the unstable ammonium bicarbonate, which further decomposes into ammonia and carbon dioxide. The indicator present in the second composition indicates the presence of ammonia to signify a positive test for urease. For example, in one embodiment the indicator can be a pH indicator that changes color when the pH of its environment is increased.

Besides both compositions being spaced apart on a single container or platform as shown in Figure 1, however, it should be understood that the first and second compositions of the present invention can be maintained in any suitable separated state prior to testing. In this regard, the first composition and the second composition can be maintained in separate containers if desired.

The ingredients that can be contained in the first composition and the second composition in accordance with the present invention will now be described in greater detail. As described above, the first composition is generally a dry or moisture-free composition containing urea in a powdered state. Urea has the chemical formula H₂NCONH₂ and is a naturally occurring product of protein metabolism. When contacted with urease, urea hydrolyzes to form unstable ammonium bicarbonate, which further decomposes into ammonia and carbon dioxide.

Urea in a powdered state for use in the present invention is available from various commercial sources. The particle size of the urea contained in the first composition is generally not critical although smaller particle sizes work more efficiently. In this regard, if desired, the urea can be ground to have a mean particle size of less than about 0.01 mm. It should be understood, however, that even smaller particle sizes may be used. For example, in one embodiment, the urea particles can have a mean particle size of less than 3 microns, and particularly less than 1 micron. By reducing the particle size, more surface area of urea is available for reaction with urease and the urea will better stick to the biopsy sample.

When using relatively smaller particles, the urea particles can have a particle size distribution such that no particles present have a size greater than about 100 microns, particularly no greater than about 10 microns, and more particularly no greater than about 5 microns. The particle size of the urea can be determined using any suitable method, such as by using transmission electron microscopy (TEM). When using transmission electron microscopy, the average diameter of each particle is measured, followed by calculating the mean diameter of the urea particles in a particular group. The average diameter of each particle can be calculated by taking the average of the smallest diameter of the particle and the largest diameter of the particle. Besides transmission electron microscopy, light scattering can also be used to determine particle sizes. The mean particle size of the urea particles In a particular group Is calculated by adding the sizes of the particles together and dividing by the number of particles.

Besides containing urea, the first composition can also contain various other dry additives. For example, in one embodiment, if desired, an anti-caking agent can also be contained within the first composition. The anti-caking agent will prevent the fine urea powder from clumping or "caking". Any suitable anti-caking agent can be used in the present invention. For example, in one embodiment, fine silicon dioxide or fine sodium alumino silicate powder can be contained in the first composition. The weight per weight (w/w) ratio of urea/silicon dioxide contained in the first composition can be any ratio from 1/1 to 100/1. The particle size of the anti-caking agent can vary depending upon the particular application. For instance, in one embodiment, the particle size of the anti-caking agent is no greater than the particle size of the urea.

The second composition, which is maintained separate from the first composition, contains an indicator for indicating the presence of ammonia. In general, any suitable indicator can be present in the second composition. In one embodiment, a pH indicator can be used that indicates a change in pH. For example, various pH indicators are available that change color as the pH is increased.

In general, when using a pH indicator, the pH of the second composition should be less than about 6.5. More particularly, the second composition can have a pH that is consistent with mammalian tissue, which typically has a pH of about 6.5.

In this regard, the pH of the second composition should be from 4.0 to 6.5, and particularly from about 4.5 to about 6.0. In this manner, when the second composition is contacted with the biopsy sample containing urea, the pH of the second composition will increase if the urea is being converted into ammonia. This rise in pH will then cause the pH indicator to signify a positive reading, such as by changing color.

The pH of the second composition should be adjusted to have a pH of from about 0.5 pH unit to about 2 pH units lower than that necessary for a color change to occur.

Consequently, when using a pH indicator, the indicator should undergo a color change or otherwise signify a positive reading when the pH of the second composition rises above neutral, and particularly above about 7.5. pH indicators useful in the present invention include indicators that undergo a change in color over a pH range of from about 5.5 to about 9.0, and particularly from about 6.5 to about 8.5.

One particular pH indicator that can be used in the present invention is phenol red. Phenol red changes from a yellow color to a red color as the pH of its surroundings increase. Phenol red is also referred to as phenolsulfonphthalein.

Other pH indicators that may be used in the present invention include p-nitro-phenol, bromthymol blue (dibromthymoisulfonph-thalein), neutral red (2-methyl-3-amino-6-dimethylaminophenazine), quino-line blue (cyanine), cresol red (o-cresolsulfonphthalein), matacresol purple (m-cresolsulfonphthalein), thymol blue (thymolsulfonphthalein), bromocresol purple (4,4'-(3H-2,1-benzoxathiol-3-ylidene)bis[2-bromo-6-methylphenol] S,S-dioxide), chlorophenol red, bromocresol green (4,4'-(3H-2,1-benzoxathiol-3-ylidene)bis[2,6-dibromo-3-methylphenol] S,S-dioxide), and bromophenol blue (4,4'-(3H-2,1-benzoxathiol-3-ylidene)bis[2,6-dibromophenol] S,S-dioxide).

A combination of indicators can be used, such as described in U.S. Patent No. 5,439,801 to Jackson. For example, methyl red can be combined with bromthymol blue.

The second composition can be made up entirety of the indicator or can Include other ingredients as desired. For example, the indicator can be present In a gel-like material. In this regard, the indicator can be combined with a gelling agent so that the second composition Is in a semi-solid state under ambient conditions.

The gelling agent can be agar. Agar is a polysaccharide complex that Is extracted from agarocytes of certain algae. Agar is available from various commercial sources. For most applications, the agar or any other gelling agent used should be nonnutritive, i.e., does not support the growth of microorganisms.

Besides, or in addition to, a gelling agent, an indicator can also be combined with a pH adjuster to maintain the pH of the second composition within preset limits. The addition of a pH adjuster is particularly beneficial when using a pH indicator to prevent against false readings. For example, as discussed above, the pH of the second composition should be from about 4.0 to about 6.5, when using a pH indicator. A suitable pH adjuster can be used to maintain the pH of the composition within this range. pH adjusters suitable for this test include acids and buffering agents. The use of a pH adjuster depends upon the make-up of the second composition and the requirements of the test. For example, reduction of the amount of buffer in the second composition leads to a much faster reaction and a faster change in colors by the indicator, with the most rapid reaction rate occurring in the absence of a buffering agent, as compared to the reaction rate when using a large amount of a buffering agent. Thus, if a high reaction rate is required, the use of buffering agents as pH adjusters should be limited.

In general, any suitable pH adjuster can be used, depending upon the requirements of the test and the second composition, including the use of acids and buffering agents such as sodium citrate, phosphate-citrate, citric acid, sulfamic acid, sodium bisulfate, sodium acetate, sodium phosphate, and potassium phosphate.

Another ingredient that may be contained in the second composition is a bactericide or a bacteristat. The bactericide or bacteristat can be used to act as a preservative for any of the other ingredients or can be used to substantially inhibit the growth of other organisms to prevent against false readings. Bactericides that can be used in the present invention include sodium azide, methyl paraben (methyl p-hydroxybenzoate), and propyl paraben (propyl p-hydroxybenzoate).

The amount of each ingredient added to the second composition will depend upon the various circumstances and the desired result. Besides maintaining the indicator as a liquid or in a gel state, the indicator can also be contained in an absorbent substrate, such as a substrate made from pulp fibers including cardboard or paper. In this embodiment, the second composition can be dry and relatively moisture free. When using a paper substrate, however, extra water and distilled water, may need to be added to the second composition in combination with the biopsy sample in order to provide enough moisture to activate the indicator.

The following is an example of one formulation that can be used as the second composition in the system of the present invention. The pH of the solid gel will be between 4 and 6.5 and particularly between 4.5 and 6.0.

| **Ingredient** | **Amount** |
|---|---|
| Agar (Extra Pure Grade) | 1.0-50.0g |
| Citric Acid | 0.001-1.0g |
| Phenol Red | 0.001-2.0g |
| Methylhydroxy Benzoate | 0.01-100.0g |
| Distilled Water | remainder |

When forming a one liter batch of the above composition, the ingredients can be added in the following amounts.

| **Ingredient** | **Reference** | **Amount** |
|---|---|---|
| Agar (Extra Pure Grade) | Merck Catalog #1.01615.9025 | 15.0g |
| Citric Acid | Merck Catalog #1.00247.1000 | 0.0145g |
| Phenol Red | Merck Catalog #1.07241.0025 | 0.110g |
| Methyl Paraben | Merck Catalog #1.06757.5000 | 2.0g |
| Distilled Water | - | 1000mL |

In producing the above gel composition, the distilled water is first heated to 95 °C. The phenol red powder is added while stirring the distilled water, and the agar is added in small amounts while the mixture is maintained at 95 °C. The citric acid and methyl paraben are then added to the mixture. The bulk liquid is cooled to 50 °C and dispensed in an amount of 0.2 mL into the second well of the present invention.

The first well of the container can contain 5 to 50 mg of the first composition, and optimally 30 mg of the resulting fine powder mixture. In the preparation of the urea mixture of the first composition for use in the first well, crystalline extra pure urea (Merck Catalog #1.08488.5000) Is mixed with silicon dioxide (Sigma Catalog #S-5631) at a weight-to-weight ratio from 1:1 to 100:1, and In one embodiment in a weight-to-weight ratio of 2:1. The mixture is subject to grinding until a fine powder mixture results.

Another helpful for understanding the present invention is illustrated In Figure 4. Instead of containing two separate wells and two separate compositions, the urease testing device contains a single composition in a dry powdered state. Specifically, In this embodiment, the urease indicating composition contains dry powdered urea combined with a dry powdered Indicator.

For example, as shown in Figure 4, a urease testing device generally 110 includes a single well 112 covered by a peelable plastic film 120. The well 112 includes a urease indicating composition 116, which contains a powdered mixture of urea and an indicator.

The powdered urea contained within the well can be a urea as described above having an average particle size of less than about 0.1 mm, particularly less than about 0.05 mm, and more particularly less than about 0.01 mm. Combined with the powdered urea is a dry or powdered Indicator, such as a pH indicator. In general, any suitable dry indicator can be present In the composition, such as any of the above- described indicators. The amount of indicator contained within the composition will generally depend upon the particular indicator chosen. Specifically, the indicator should be present in the composition in an amount sufficient to show a color change when the composition Is contacted with urease present in a biopsy sample.

The biopsy sample is placed in the well and mixed with the powdered composition. Any moisture present in the biopsy sample can be used to activate the urea and the indicator. If necessary, however, an aqueous solution, such as distilled water, can be added with the biopsy sample. If urease is present in the biopsy sample, the urease will convert the urea into ammonia which, in turn, will cause the indicator to indicate a positive result, such as by changing color.

An anti-caking agent as described above is also contained in the dry powdered composition. However, a pH adjuster or a bactericide will most likely not be needed, although both ingredients can be contained In the composition if desired.

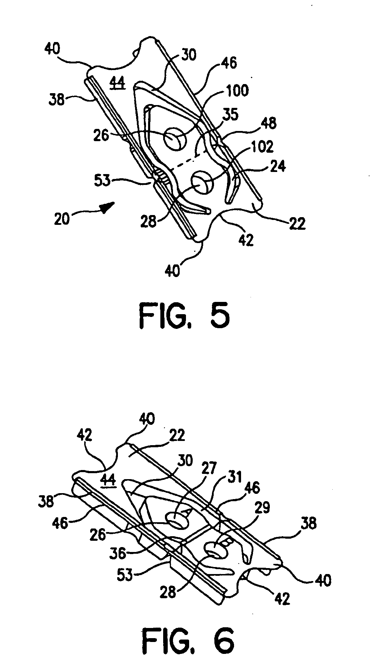

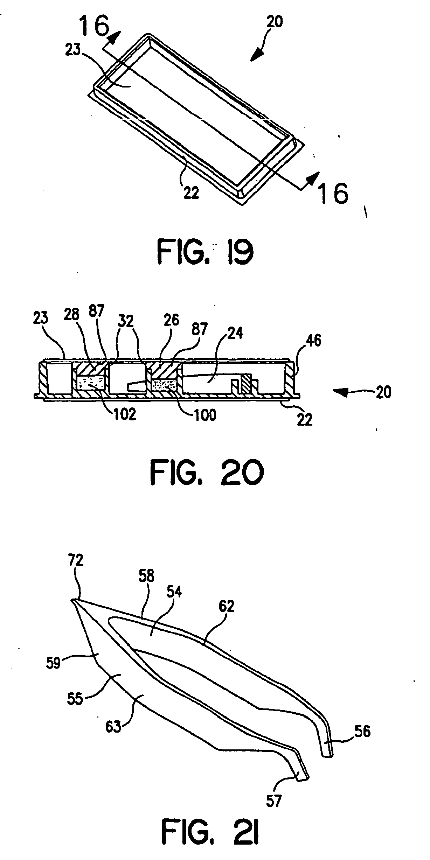

Figure 5 discloses a diagnostic system 20 that may be utilized for many types of diagnostic testing. Such diagnostic tests utilize a biological test specimen such as, for example, tissue biopsy, blood or saliva. The diagnostic system 20 may include a container 22 and a mechanism by which a user may manipulate a sample of tissue, such as, for example, the specimen-handling tool 24 that is shown In Figures 5, 10 and 14. As depicted in Figure 19, the diagnostic system 20 may further include an overlying member 23.

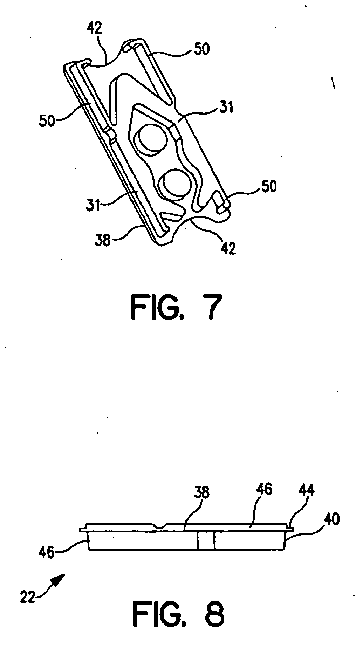

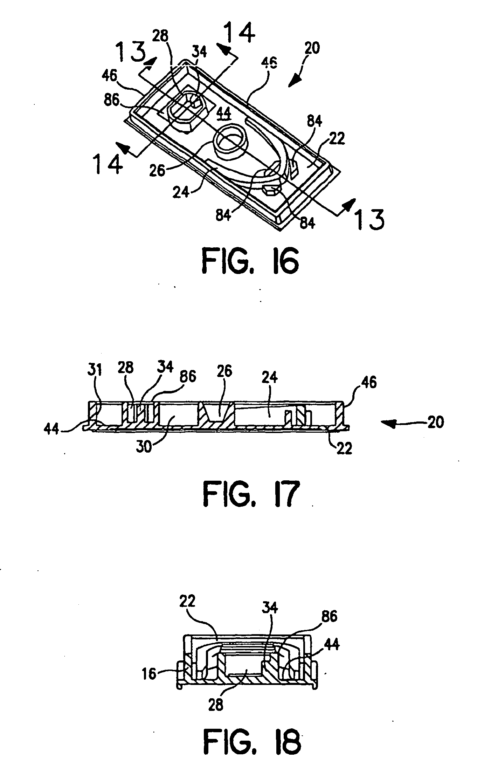

As shown in Figures 5-7, 9, and 16, the container 22 may include a first well 26 and a second well 28. The wells 26 and 28 may be defined, at least in part, by the walls 27 and 29, respectively. The wells 26 and 28 may be formed to have a variety of different depths and cross-sectional shapes, some variations of which are shown In Figures 5, 16-18 and 20. The wells 26 and 28 of the container 22 may be variously formed, and may have similar configurations or dissimilar configurations. As shown in Figures 7 and 17, the wells 26 and/or 28 may be generally frustoconical in shape, although the wells 26 and/or 28 may be cylindrical or otherwise shaped. The wells 26 and/or 28 may be formed so that, when viewed from the top of the container 22, the wells 26 and/or 28 have a non-circular shape, such as an elliptical, square, rectangular, D-shaped or any other shape.

One or more projecting members, such as the projecting member 34 that is shown in Figures 16-18, may be disposed within one or both of the wells 26 and 28. At least a portion of the projecting member 34 may be disposed outside of the interior of the wells 26 and/or 28. The projecting member 34 may be integrally formed with the walls 27 and 29, or may be attached to the walls 27 and/or 29. Such projecting members 34 may be configured to assist removal of the specimen such as, for example, a biopsy specimen, from the specimen-handling tool 24. These projecting members 34 may be configured to assist the user in accurately positioning a specimen within the well 26 or 28.

The wells 26 and 28 may also include a step such as the step 32 that is depicted in Figure 20.

The container 22 may have many different overall exterior shapes, such as, for example, the generally rectangular shape as shown in Figures 5, 6 and 9. The container 22 may be alternately shaped, such as, for example, square, oblong, triangular, and the like. The container 22 may, as shown in Figures 5-7, include two elongated sides 38, two ends 40 and a surface 44. The ends 40 may be configured to be easily grasped by a user and one, none or both of the ends 40 may include an arcuate portion 42 as shown in Figures 5 - 9.

As shown in Figures 5, 6, 8 and 9, the container 22 may include a surface 44. The first and/or second wells 26 and 28, respectively, may be configured to extend downwardly from the surface 44. As shown in Figures 5 and 6, the container 22 may also include a cavity 30. In a similar manner, the cavity 30 may be configured to extend downwardly from the surface 44, as shown in Figures 5, 6 and 9. As shown in Figures 16-18, one or both of the wells 26 and 28 and/or the cavity 30 may be formed so as to extend upwardly from at least a portion of the surface 44.

A mechanism by which a user may manipulate a sample of tissue, such as, for example, the specimen handling tool 24 such as that shown in Figures 5 and 10-15, may also be included in particular embodiments of the diagnostic system 20 of the present invention. The specimen-handling tool 24 may be disposed within the cavity 30.

The cavity 30 may, as shown in Figures 5-7, be configured so that it is disposed about at least a portion of one of the first and/or second wells 26 and 28, respectively. The container 22 may also be configured so that a specimen handling tool 24 may be otherwise retained in the container 22 so that it is disposed about at least a portion of one of the first and/or second wells 26 and 28, respectively. The specimen handling tool can be shaped like a pair of tweezers as shown or in the shape of a single member pointed instrument that can pick up a specimen by lancing the sample. As shown in Figures 16 and 17, the container 22 may be configured so that the specimen-handling tool 24 is secured in a particular position by one or more ribs 84. The specimen-handling tool 24 may be removably attached to the container 22 by one or more locking arms, breakaway tabs, adhesive, or the like.

One or more rails 46 may be included in selected embodiments of the present invention and may be disposed on the container 22 so that the rails extend upwardly along at least a portion of the surface 44. One or more rails 46 may also be configured to extend outwardly from the container 22. At least one gap 48 may be formed in one of the rails 46 that extend along a portion of the container 22.

As shown in Figure 7, one or more supports 50 may be provided which extend downwardly from the surface 44. As seen in Figure 7, the supports 50 may be attached to the wall (or walls) 31 that form at least a portion of the cavity 30. The supports 50 may extend outwardly from the wall 31 to permit the container 22 to rest in a stable position on a horizontal or other surface. The rails 46 and the supports 50 may be configured to enable the container 22 to be automatically processed through a variety of equipment.

If desired, the surface 44 may be configured so that various indicia, such as letters, numbers, symbols and other characters, may be placed onto or formed into the surface 44. For example, and as shown In Figure 6, each well 26 and/or 28 may be given a particular designation, such as A or B, and that designation may be printed upon the surface 44.

The container 22 may be formed from a variety of materials, including, for example, polycarbonate, polystyrene, polypropylene, polyethylene, polyvinylchloride, or any other type of polyolefin.

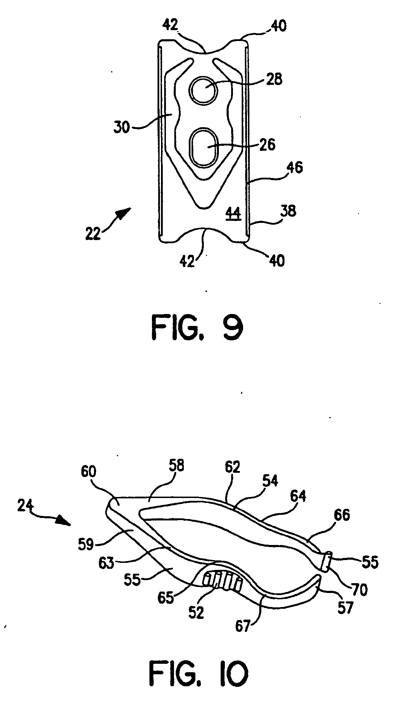

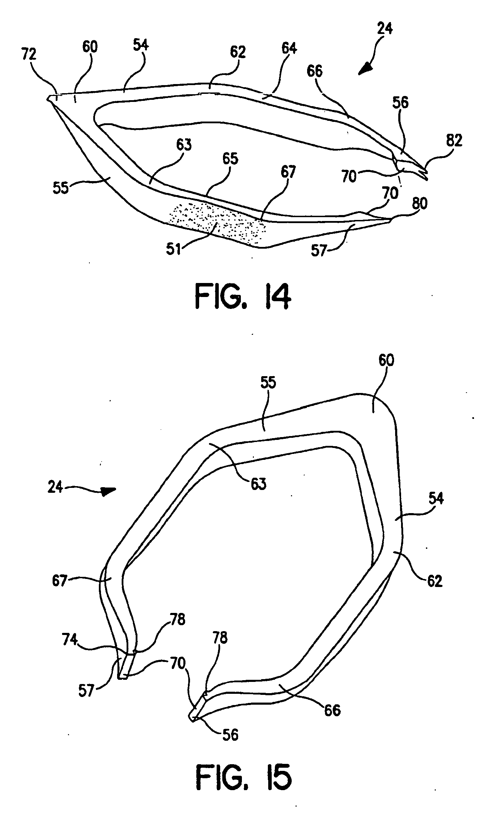

Particular examples of the specimen-handling tool 24 are shown in Figures 10 - 15 and 21. The specimen-handling tool 24 may include, as shown in Figures 10-13, a pair of cooperating arms 54 and 55. Each arm 54 and 55 may Include a tip portion 56 and 57, respectively. The arms 54 and 55 may each also include a rear portion 58 and 59, respectively. The arms 54 and 55 may be joined to each other at their rear portions 58 and 59, respectively, forming a joined end 60. The joined end 60 may be configured to assist the user in accomplishing particular tasks, such as, for example, manipulating a specimen, removing a plug 86 from one of the first and/or second wells 26 and 28, respectively, as well as other tasks. The outermost portion of the joined end 60 may be variously configured, and may be formed as a narrow projection, such as that shown in Figure 14.

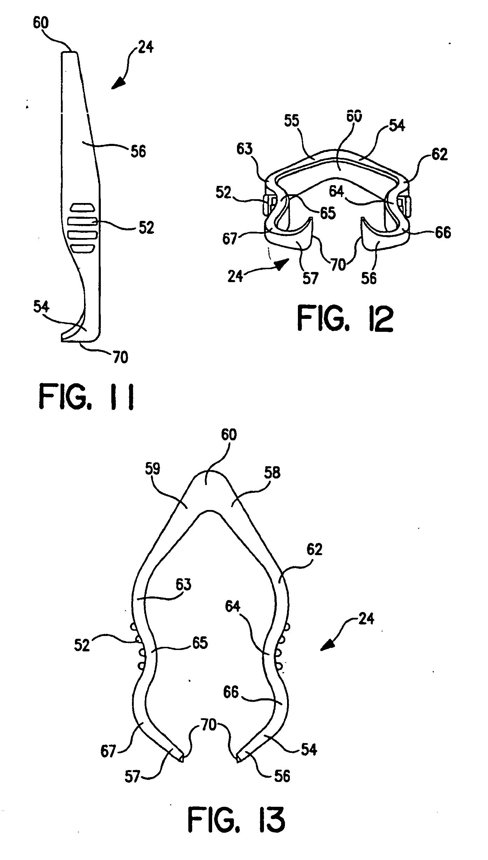

As seen in Figures 12 and 13, each arm 54 and 55 may also include a rearward arcuate portion 62 and 63, respectively, and a forward arcuate portion 66 and 67, respectively. Disposed between each rearward arcuate portion 62 and 63 and its corresponding forward arcuate portion 66 and 67, respectively, is an intermediate arcuate portion 64 and 65, respectively. The arcuate portions 62-64-66 and 63-65-67 of each arm 54 and 55, respectively, may be configured so that the area disposed between the arms 54 and 55 is approximately hourglass in shape. In such an embodiment, the rearward arcuate portions 62 and 63 and forward arcuate portions 66 and 67 curve outwardly, and the intermediate arcuate portions 64 and 65 curve inwardly.

The intermediate arcuate portions 64 and 65 may be formed so that a user may more easily grip these portions. As shown in Figure 10, one or more ribs 52 may be positioned on the outer surface of the intermediate arcuate portions 64 and 65. Alternately, a portion of the arms 54 and/or 55 may have a roughened texture to enable a user to more effectively grasp and manipulate the specimen-handling tool 24, such as is shown in Figure 14 at 51.

The arms 54 and/or 55 may include fewer or more arcuate portions than the three arcuate portions described above, such as the specimen-handling tool shown in Figure 15. The arcuate portions of the arms 54 and/or 55 may have a more or less pronounced arcuate shape than what is depicted in Figure 10. For example and as shown in Figures 14 - 16 and 21, other configurations of the arms 54 and 55 may be used in the specimen-handling tool 24.

The tip portions 56 and 57 may be variously formed to enable a user to manipulate a specimen. The tip portions 56 and 57 may be formed to include a surface such as the surfaces 70. The surfaces 70 may be variously shaped and, in particular, one or both of the surfaces 70 may be curved (as shown in Figure 14) or flat (as shown in Figure 10). The surfaces 70 may be rough or smooth. Also, structures such as the ridges 78 that are depicted in Figure 15 may also be positioned on one or more of the surfaces 70. The surfaces 70 may be disposed so that they are at least somewhat facing each other, thereby enabling a user to grasp a specimen and hold it between the surfaces 70. As shown in Figure 14, the tip portions 56 and/or 57 may curve outwardly, and may, in some embodiments such as is shown in Figure 15, end in a relatively sharp edge 74. One or both of the tip portions 56 and 57 may include a point, such as the point 80 shown in Figure 14 or a fork 82, also shown in Figure 14, or any number of other configurations.

The specimen-handling tool may be formed from a variety of materials, including, for example, polycarbonate, polystyrene, polypropylene, polyethylene, polyvinylchloride, or any other type of polyolefin.

Referring now to Figures 19 and 20, an overlying member 23 may be disposed over at least a portion of the surface 44 of the container 22. At least a portion of the cavity 30 may be formed by the wall 31. The overlying member 23 may take the form of an adhesive-backed label that adheres to at least a portion of the surface 44. The overlying member 23 may overly any combination of the first well 26, the second well 28 and the cavity 30.

The overlying member 23 may also be used to seal the first and second wells 26 and 28, respectively. In some embodiments, the overlying member may be used to regulate the rate of water vapor transmission to and from the wells 26 and 28 of the container 22. The overlying member 23 may also be configured so that, if the overlying member 23 is removed prematurely or inadvertently, it may be easily reapplied to the container 22 so that the wells 26 and 28 may be resealed.

The overlying member 23 may also be used to retain the specimen-handling tool 24 within the cavity 30. The overlying member 23 may also be configured only to retain the specimen-handling tool 24 within the cavity 30. In some embodiments, the overlying member 23 may be adhered to at least a portion of the specimen-handling tool 24 so that, when the overlying member 23 is removed form the container 22, the specimen-handling tool 24 is also removed from the container 22. Although this may be accomplished in many different ways, the intermediate arcuate portions 64 and 65 may, when the specimen-handling tool 24 is positioned within the cavity 30, be level with or rise slightly above the surface 44 so as to contact and be adhered to the overlying member 23.

As shown in Figure 20, a plug 87 may also be used to at least partially seal each well 26 and 28. In such a configuration, the overlying member 23 does not need to seal the well that contains the plug 87, but may merely be positioned above the well 26 and/or 28. The plug 87 may be formed from a variety of materials, including, for example, rubber, wax, silicone, or any of a variety of plastics. A film cover 86, shown in Figure 18, may also be applied to a portion of the container 22, such as, for example, the well 28.

The overlying member 23 may be adhered or otherwise connected to one or more of the plugs 87 so that, when the overlying member 23 is separated from the container 22, one or more of the plugs 87 may also be removed. The plug 87 may also be removed with the specimen-handling tool.

### Example

The following example was performed in order to demonstrate the stability of a urease testing device.

A test slide was prepared containing the urea composition and the indicator gel composition described above. The indicator gel composition, however, did not contain the methyl paraben bactericide or the citric acid pH adjuster.

Specifically, the gel composition contained the following:

| **Ingredient** | **Amount** |
|---|---|
| Extra Pure Grade Agar | 1.4941g |
| Phenol Red | 0.0110g |
| Distilled Water | 100.00mL |

The shelf life of the above prepared slide was then compared with the shelf life of a commercial product marketed under the name CLO-TEST by Ballard Medlcal/Kimberly Clark of Draper, Utah. The CLO-TEST product includes a urease indicator composition which contains a mixture of urea and an Indicator in a gel as described in U.S. Patent No. 4,748,113.

Three test slides were compared with three samples of the CLO-TEST product. A standardized CLO-TEST Color Chart developed prior to the experiment was used to assign numerical scores to the color of the samples during the experiment.

The slides were affixed to a polystyrene box Introduced into a chamber set at 37°C, 100% relative humidity, and 10% carbon dioxide. Photographs were taken every 24 hours for a period of 45 days, which were then assessed and given a score using the CLO-TEST Color Chart. Using color readings with scores of equal to or greater than 4 as unusable, the CLO-TEST samples were deemed unusable on day 4, while the test slides of the present invention were still viable on day 45.

The shelf life of the test slide was also tested with an artificial biopsy by means of a tissue sample containing deliberately introduced urease. The artificial biopsy sample was placed In the first well containing the powdered urea. The sample was coated with urea, and then placed In the second well containing the indicator gel composition. Observations of the color change of the gel revealed It was still viable for the detection of ammonia after 39 days, when the gel was checked.

It should be noted that any given range presented herein is Intended to include any and all lesser included ranges. For example, a range of from 45-90 would also include 50-90; 45-80; 46-89 and the like. Thus, the range of 95% to 99.999% also includes, for example, the ranges of 96% to 99.1%, 96.3% to 99.7%, and 99.91 to 99.999%.

## Claims

1. A material well suited for detecting the presence of urease in a gastric material for diagnosing a gastrointestinal disorder comprising: a composition comprising a powder, said composition including urea and an anti-caking agent, said urea having a mean particle size of less than about 0.01 mm, wherein said anti-caking agent comprises sodium alumino silicate or silica having a particle size not greater than the particle size of said urea.

2. A material as defined in claim 1, wherein said composition further comprises an indicator that is configured to indicate the presence of ammonia created when said urea contacts urease.

3. A material as defined in claim 1, wherein said composition further comprises a bactericide.

## Patentansprüche

1. Ein Material, das für das Detektieren des Vorhandenseins von Urease in einem gastrischen Material zum Diagnostizieren einer gastrointestinalen Krankheit gut geeignet ist und das umfasst: eine Zusammensetzung mit einem Pulver, wobei die genannte Zusammensetzung Harnstoff und ein Antiagglomerationsmittel umfasst, wobei der genannte Harnstoff eine mittlere Teilchengröße von weniger als ungefähr 0,01 mm besitzt, und wobei das Antiagglomerationsmittel Natriumaluminiumsilikat oder Silica mit einer Teilchengröße, die nicht größer als die Teilchengröße des Harnstoffs ist, umfasst.

2. Ein Material, wie es in Anspruch 1 definiert ist, in dem die genannte Zusammensetzung weiterhin einen Indikator umfasst, der dazu ausgebildet ist, das Vorhandensein von Ammoniak anzuzeigen, der erzeugt wird, wenn der genannte Harnstoff in Kontakt mit Urease gerät.

3. Ein Material, wie es in Anspruch 1 definiert ist, in dem die genannte Zusammensetzung weiterhin ein Bakterizid umfasst.

## Revendications

1. Matériau bien adapté pour détecter la présence d'uréase dans une matière gastrique pour diagnostiquer un trouble gastro-intestinal, comprenant : une composition comprenant une poudre, ladite composition contenant de l'urée et un agent antiagglomérant, ladite urée ayant une granulométrie moyenne inférieure à environ 0,01 mm et ledit agent antiagglomérant comprenant de l'aluminosilicate de sodium ou de la silice ayant une granulométrie ne dépassant pas la granulométrie de ladite urée.

2. Matériau selon la revendication 1, dans lequel ladite composition comprend en outre un indicateur qui est configuré pour indiquer la présence d'ammoniac créé quand ladite urée vient au contact d'uréase.

3. Matériau selon la revendication 1, dans lequel ladite composition comprend en outre un bactéricide.
